(19) Europäisches Patentamt
European Patent Office
Office européen des brevets

(11) Veröffentlichungsnummer : **0 026 343**
**B1**

# (12) EUROPÄISCHE PATENTSCHRIFT

(45) Veröffentlichungstag der Patentschrift : **22.06.83**

(51) Int. Cl.$^3$ : **C 07 D307/79**

(21) Anmeldenummer : **80105209.3**

(22) Anmeldetag : **02.09.80**

(54) **Verfahren zur Herstellung von Bis-benzofuranyl-Verbindungen.**

(30) Priorität : **14.09.79 DE 2937231**

(43) Veröffentlichungstag der Anmeldung : **08.04.81 Patentblatt 81/14**

(45) Bekanntmachung des Hinweises auf die Patenterteilung : **22.06.83 Patentblatt 83/25**

(84) Benannte Vertragsstaaten : **CH DE FR GB IT LI NL**

(56) Entgegenhaltungen :
**DE A 2 238 734**
**DE A 2 837 736**
**TETRAHEDRON LETTERS, Nr. 23 Juni 1979 Oxford, New York, Paris, Frankfurt A. HERCOUET et al. « Une nouvelle voie d'accès aux benzofurannes » Seiten 2145 bis 2148**

(73) Patentinhaber : **HOECHST AKTIENGESELLSCHAFT**
**Postfach 80 03 20**
**D-6230 Frankfurt/Main 80 (DE)**

(72) Erfinder : **Schinzel, Erich, Dr.**
**Ostpreussenstrasse 43**
**D-6238 Hofheim am Taunus (DE)**

**EP 0 026 343 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von Bis-benzofuranyl-Verbindungen

Es ist bereits vorgeschlagen worden, Benzofuran-Verbindungen durch Cyclisierung von o-Acyloxybenzyl-phosphoniumsalzen herzustellen. So wird z. B. in der DOS 2 837 736 ein entsprechendes Verfahren beschrieben, bei welchem als Cyclisierungsmittel Basen aus der Gruppe der Hydroxide und der Alkoholate von Alkalimetallen, wie Natrium-t-amylat und Kalium-t-butylat, bzw. Natriumhydroxid und Kaliumhydroxid zur Anwendung kommen. Versuche, Bis-benzofuran-Derivate unter den in obiger Offenlegungsschrift genannten Bedingungen herzustellen, scheiterten ; es wurden nur stark verfärbte, nicht fluoreszierende und größtenteils alkalilösliche Reaktionsprodukte erhalten.

Aus Tetrahedron Letters 23, Juni 1979 S. 2145-2148 ist auch bekannt, Benzofuran-Derivate herzustellen durch Umsetzung von o-Hydroxy-benzyl-triphenylphosphonium-bromiden mit organischen Säurechloriden. Dieses Verfahren wird in Gegenwart von Triäthylamin in Toluol durchgeführt, d. h. in homogener Phase, wobei zwingend 3 Mol Triäthylamin auf eine Phosphoniumgruppe benötigt werden.

Es wurde nun demgegenüber gefunden, daß man die Cyclisierung der O-Acyl-Verbindungen der Hydroxy-benzylphosphonium-halogenide in heterogener Phase mit schwächeren Basen durchführen kann, die zudem auch noch in wesentlich geringeren Mengen benötigt werden.

Gegenstand der Erfindung ist somit ein Verfahren zur Herstellung von Bis-[benzofuranyl-(2)-] Verbindungen der allgemeinen Formel (I),

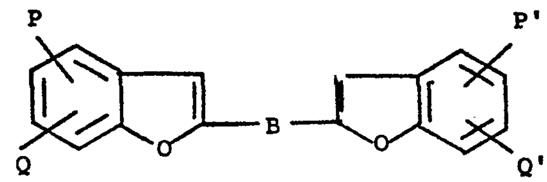

(I)

in der B eine direkte Bindung oder eine der nachstehend aufgeführten Gruppen bedeutet

$-CH{=}CH-$ , 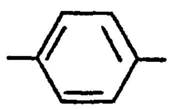 , 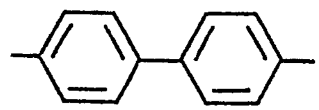 ,

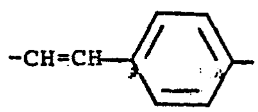 , 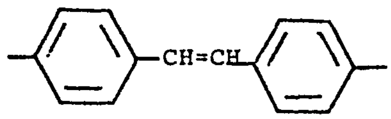 ,

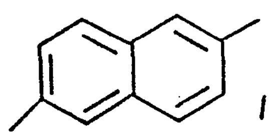 , 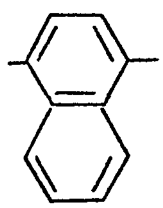 und 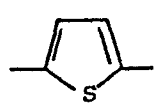

P, Q, P′ und Q′ unabhängig voneinander Wasserstoff oder Halogenatome, niedere Alkoxy- oder Phenylgruppen, gegebenenfalls funktionell abgewandelte Carboxylgruppen, oder P und Q sowie P′ und Q′ zusammen einen ankondensierten Benzolkern bedeuten, indem man

Verbindungen der allgemeinen Formel (II)

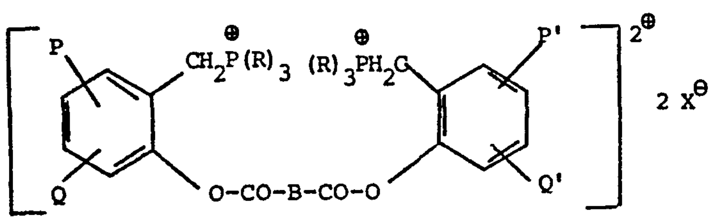

(II)

in welcher

B, P, Q, P′, Q′ die oben angegebene Bedeutung haben und

R einen Alkyl-, Aryl- oder Aralkylrest und

X ein Anion bedeuten, in durch Alkylgruppen oder Halogenatome substituierten, aromatischen

Kohlenwasserstoffen und in Gegenwart von mindestens 2 Mol Alkali- oder Erdalkalicarbonaten bei einer Temperatur von 100 bis 200 °C unter Abdestillieren des aus den Carbonaten gebildeten Wassers cyclisiert.

Als Carbonate verwendet man vorteilhaft Natrium- oder Kaliumcarbonat. Es erweist sich als vorteilhaft, diese Kondensationsmittel in getrocknetem, wasserfreiem Zustand einzusetzen. Per Mol der Verbindungen (II) werden 2 Mol Alkalicarbonat verwendet, ein Überschuß bis zur doppelten molaren Menge schadet nicht.

Als Lösungsmittel verwendet man wasserfreie, aromatische Kohlenwasserstoffe wie Toluol, Xylole, Xylol-Gemische, Chlorbenzol, o-Dichlor-benzol, 1,2,4-Trichlorbenzol, Trichlorbenzol-Gemische, α-Methyl-naphthalin, Gemische aus α- und β-Methylnaphthalin, α-Chlor-naphthalin und Tetralin. Bevorzugt werden chlorierte Kohlenwasserstoffe wie Chlorbenzol, o-Dichlorbenzol und Trichlorbenzol verwendet. In diesen Lösungsmitteln sind die Verbindungen (II) und die als Cyclisierungsmittel verwendeten Carbonate nur sehr wenig löslich, so daß die Reaktion im wesentlichen heterogen abläuft. Auch die Reaktionsprodukte (I) besitzen in den angewandten Lösungsmitteln im allgemeinen nur eine geringe Löslichkeit, sie lassen sich daher durch Absaugen und Auswaschen in einfacher Weise isolieren und rein darstellen.

Die optimale Reaktionstemperatur schwankt in Abhängigkeit von dem Mittelglied B und den Substituenten P, Q, P', Q' zwischen 100 und 200 °C, die bevorzugte Temperatur liegt zwischen 130 und 180 °C. Es ist zweckmäßig, bei der Siedetemperatur des gewählten Lösungsmittels unter Verwendung eines Wasserabscheiders zu arbeiten. Unter diesen Bedingungen wird das aus den Carbonaten gebildete Wasser laufend ausgetragen und kann so nicht zur Aufspaltung der Esterbindung in den Ausgangsverbindungen (II) beitragen. Zur Unterstützung dieses Vorgangs und auch um die Verbindungen (I) und (II) vor Oxydationsprozessen zu schützen ist es vorteilhaft, über das Reaktionsgemisch einen schwachen Strom von getrocknetem Stickstoff zu leiten.

Die Verbindungen der allgemeinen Formel (II) erhält man in an sich bekannter Weise auf dem folgenden Reaktionsweg durch Seitenkettenhalogenierung der Bis-o-kresyl-ester (IV) zu den Bisbenzylhalogeniden (III) und deren Umsetzung mit Phosphinen $P(R_3)$.

In diesen Formeln haben die Reste B, P, Q, P', Q' und R die eingangs erwähnte Bedeutung, X bedeutet Halogen.

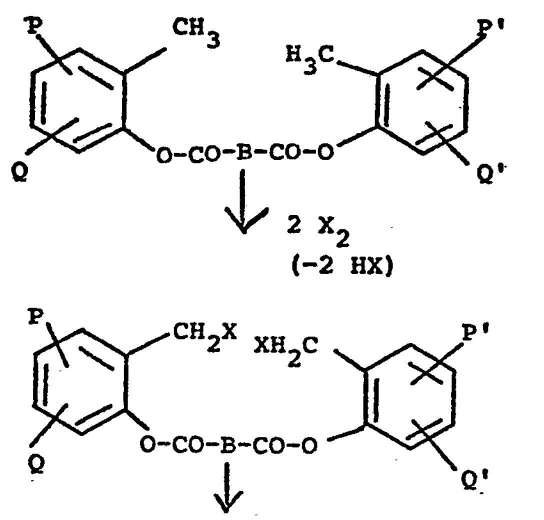


(IV)

(III)

(II)

Bei der Wahl geeigneter Lösungsmittel und Reaktionsbedingungen gelingt es auch, zwei oder mehrere Stufen des vorstehenden Syntheseweges und die erfindungsgemäße Cyclisierung der Verbindungen (II) in einem « Eintopfverfahren » zu vereinen. So können beispielsweise die Bis-benzylhalogenide (III) in o-Dichlorbenzol mit Triphenylphosphin zu den Bis-phosphoniumsalzen (II) umgesetzt und diese ohne Zwischenisolierung nach Zugabe von 2 Mol Natriumcarbonat erfindungsgemäß zu den Verbindungen (i) cyclisiert werden.

Durch das erfindungsgemäße Verfahren eröffnet sich ein technisch einfacher Weg zur Herstellung von Bis-[benzofuranyl-(2)]-Verbindungen, die wertvolle optische Aufheller und Aufhellervorprodukte darstellen für mehrere Einsatzgebiete in der Textil- und Waschmittelindustrie.

Bei den in den vorstehenden Formeln unter P, Q, P' und Q' angegebenen Definitionen sind unter einer funktionnell abgewandelten Carboxygruppe zunächst deren Salze mit farblosen Kationen zu verstehen, wobei Alkalimetall-oder Ammoniumionen bevorzugt sind, und weiterhin solche funktionellen Derivate einer Carboxygruppe, von deren Kohlenstoffatom drei Bindungen zu Heteroatomen ausgehen, insbesondere die Cyangruppe, eine Carbonestergruppe oder eine Carbonsäureamidgruppe. Unter

Carbonsäureestergruppen sind insbesondere solche der allgemeinen Formel $COOR^1$ zur verstehen, in welcher $R^1$ einen Phenylrest oder eine gegebenenfalls verzweigte niedere Alkylgruppe darstellt, wobei diese Reste weitere Substituenten, wie eine Alkoxygruppe enthalten können. Unter einer Carbonsäureamidgruppe ist insbesondere eine solche der Formel $CONR^2R^3$ zu verstehen, in welcher die Reste $R^2$ und $R^3$ Wasserstoffatome oder niedere, gegebenenfalls substituierte Alkylgruppen bedeuten, die auch gemeinsam mit dem Stickstoffatom einen hydroaromatischen Ring bilden können, ferner Säurehydrazide und die analogen Thioderivate. Unter dem Begriff « niedere » sind solche Gruppen zu verstehen, die 1-4 C-Atome enthalten.

Nach dem erfindungsgemäßen Verfahren können z. B. die folgenden Bis-[benzofuranyl-(2)]-Verbindungen erhalten werden :

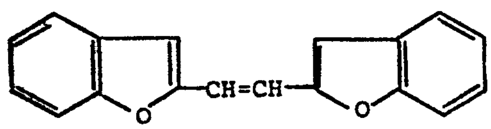

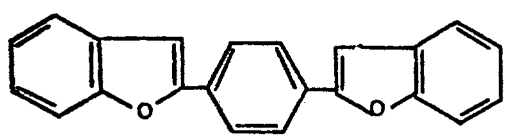

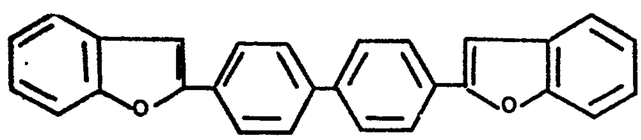

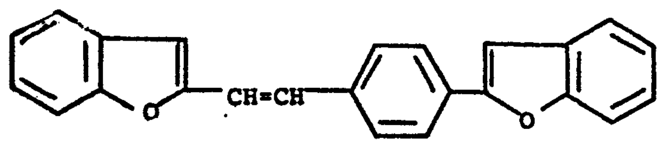

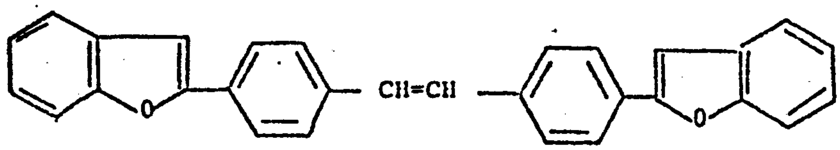

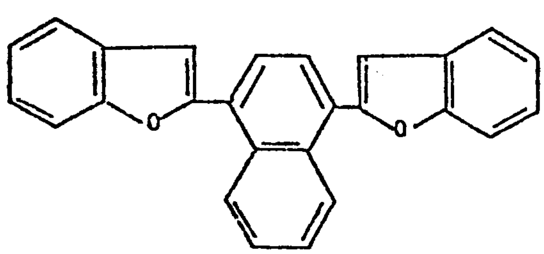

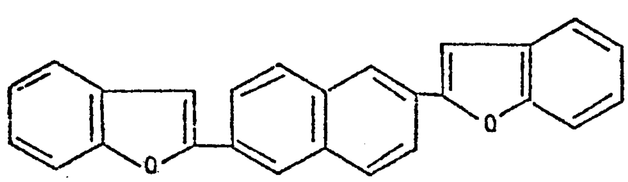

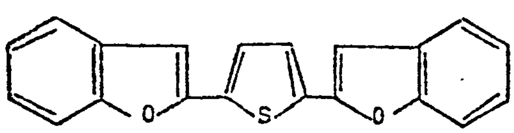

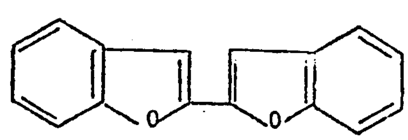

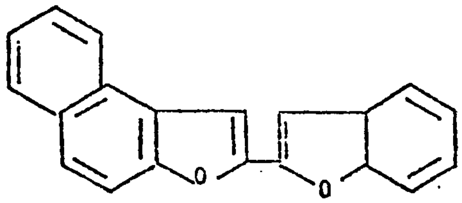

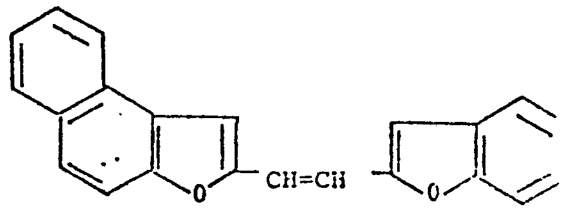

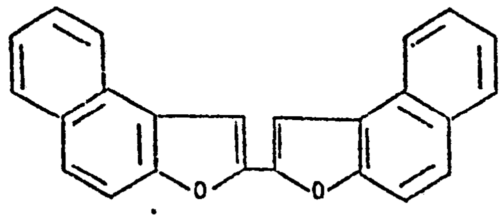

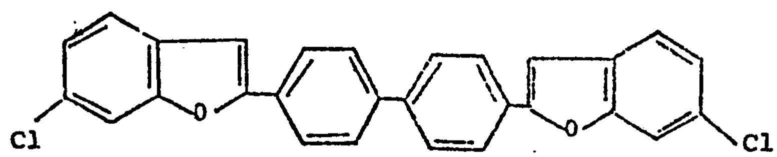

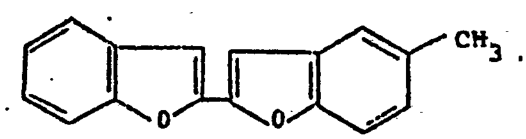

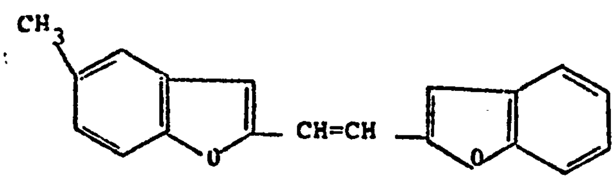

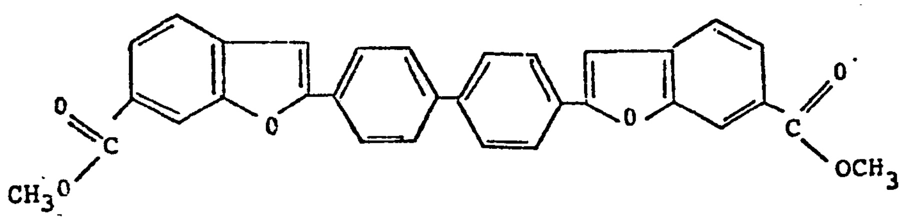

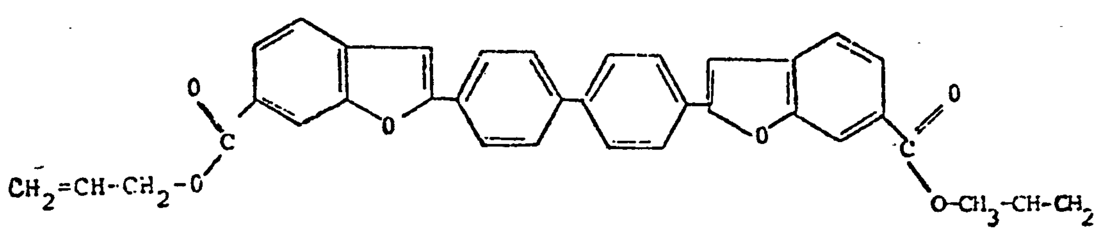

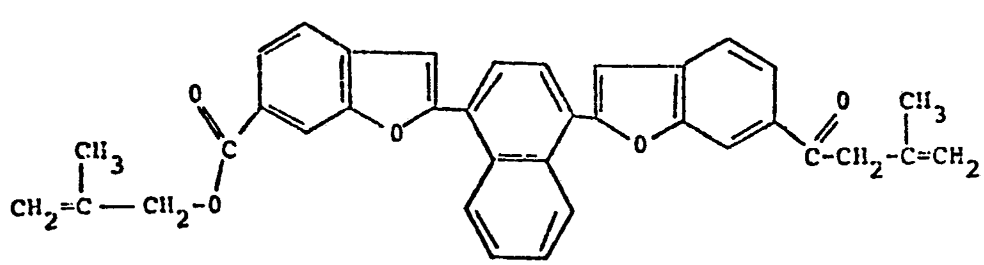

Beispiel 1

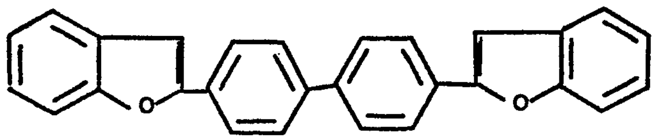

(1)

27,62 g des Bis-phosphoniumsalzes (2) werden in 60 ml o-Dichlorbenzol eingetragen und nach Zugabe von 8,6 g Kaliumcarbonat (getrocknet, gepulvert) unter guter Rührung und unter Überleiten eines schwachen Stickstoffstromes zum Sieden erhitzt. Man kocht 4 Stunden am Wasserabscheider, läßt auf Raumtemperatur erkalten und saugt ab. Das Nutschgut wird mehrmals mit Methanol gedeckt und mit Wasser neutral gewaschen. Nach dem Trocknen bei 60° i. V. werden 7,34 g des 4,4'-Bis-[benzofuranyl-(2)]-biphenyls (1) vom Schmp. 350-360° als gelbliches Kristallpulver erhalten. Das entspricht einer Ausbeute von 76 % der Theorie. In gleicher Ausbeute wird (1) bei Verwendung wasserfreiem Natriumcarbonats erhalten.

Das Bis-phosphoniumsalz (2)

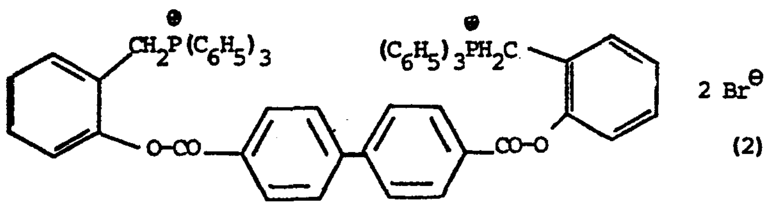

(2)

wird auf folgendem Wege hergestellt : 58 g der Bis-brommethyl-Verbindung (3) werden unter Rührung in 500 ml o-Dichlorbenzol eingetragen und mit 57,7 g Triphenyl-phosphin versetzt. Bei 90° entsteht zunächst eine klare Lösung, aus welcher sich nach kurzer Zeit das Phosphoniumsalz abscheidet. Man rührt 3 Stunden bei 95-100° nach, läßt auf Raumtemperatur erkalten und saugt ab. Das Nutschgut wird mehrmals mit o-Dichlorbenzol und Toluol gewaschen und bei 60° im Vakuum getrocknet. Es werden 107,7 g der Bis-phosphonium-Verbindung (2) als farbloses Pulver vom Schm. 259-264° erhalten, entsprechend 97,5 % der Theorie.

Die Bis-brommethyl-Verbindung (3)

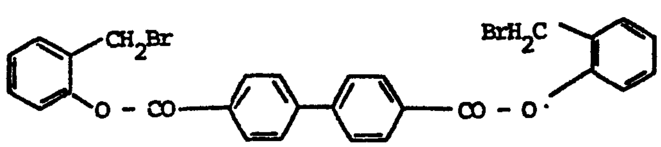

(3)

wird wie folgt hergestellt : 148 g des Diphenyl-dicarbonsäure-di-kresylester (4) werden unter Rührung in 1,5 l Tetrachlorkohlenstoff in der Siedehitze gelöst. Nach dem Erkalten auf 60° werden 124,6 g N-Bromsuccinimid und 1,4 g Azo-iso-buttersäuredinitril zugesetzt und das Reaktionsgemisch erneut zum Sieden erhitzt. Unter UV-Bestrahlung wird 1 Stunde am Rückfluß gekocht, anschließend auf 5° abgekühlt und abgesaugt. Das Nutschgut wird zweimal mit Tetrachlorkohlenstoff gewaschen und im vakuum bei 60° getrocknet. Das erhaltene farblose Pulver wird bei Raumtemperatur mit 1,5 l Wasser verrührt, abgesaugt mit Wasser gewaschen und erneut getrocknet. Es werden 157,9 g der rohen Verbindung (3) als hellgelbes Pulver vom Schmp. 182,5-191° erhalten, entsprechend 78 % der Theorie. Nach Umlösung aus Toluol schmilzt die Bis-brommethyl-Verbindung (3) bei 190,5-194 °C.

Zur Herstellung des Bis-o-kresylester der Diphenyl-dicarbonsäure (4)

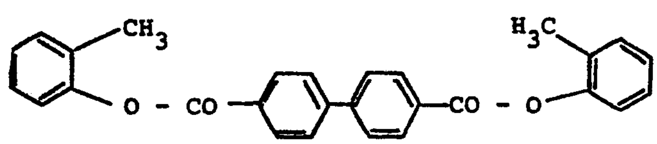

(4)

werden 139,5 g des Diphenyl-dicarbonsäurechlorids bei Raumtemperatur in 250 ml o-Dichlorbenzol eingetragen und unter Rühren mit 105 ml o-Kresol versetzt. Unter Überleiten von Stickstoff erhitzt man im Verlauf von 30 Minuten zum Sieden und kocht 2 Stunden am Rückfluß, bis kein Chlorwasserstoff mehr nachweisbar ist. Das Reaktionsgemisch wird abgekühlt, mit 350 ml Ethanol verdünnt und 1 Stunde bei + 5° nachgerührt. Man saugt ab, wäscht das Nutschgut mehrmals mit Ethanol und trocknet bei 60° im Vakuum. Es werden 200,7 g der Verbindung (4) vom Schmp. 152-154,5° erhalten, entsprechend einer Ausbeute von 95 % der Theorie.

Beispiel 2

58 g der Bis- brommethyl-Verbindung (3) werden bei Raumtemperatur in 500 ml o-Dichlorbenzol eingetragen und unter Rühren mit 57,7 g Triphenylphosphin versetzt. Man erhitzt auf 100° und hält 3 Stunden bei dieser Temperatur. Anschließend werden bei 100° 21,2 Natriumcarbonat (getrocknet, gepulvert) zugegeben und das Reaktionsgemisch unter einem schwachen Stickstoffstrom zum Sieden erhitzt. Man kocht 4 Stunden am Wasserabscheider, läßt abkühlen und saugt bei Raumtemperatur ab. Das Nutschgut wird mehrmals mit Methanol gedeckt und mit Wasser neutral gewaschen. Nach dem Trocknen bei 60° im Vakuum werden 29,6 g des 4,4'-Bis-[benzofuranyl-(2)]-biphenyls (1) vom Schmp. 356-360° als hellgelbes Pulver erhalten. Das entspricht einer Ausbeute von 76,7 % der Theorie bezogen auf die Ausgangsverbindung (3).

**Ansprüche**

1. Verfahren zur Herstellung von Bis-[benzofuranyl-(2)]-Verbindungen der allgemeinen Formel (I)

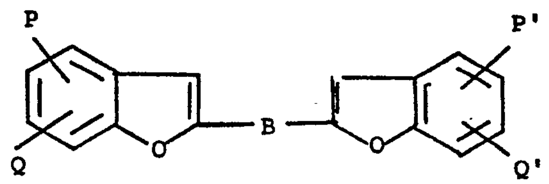

(I)

in welcher B eine direkte Bindung oder eine der nachstehend aufgeführten Gruppen bedeutet

$-CH=CH-$ , 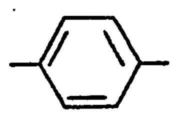 , 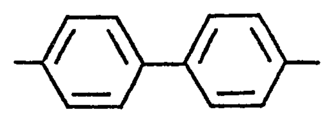 ,

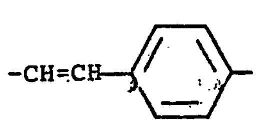 , 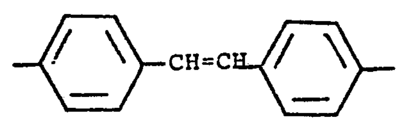 ,

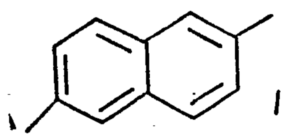 , 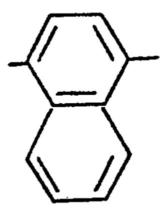 und 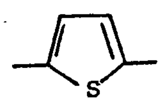

P, Q, P' und Q' unabhängig voneinander Wasserstoff oder Halogenatome, niedere Alkoxy- oder Phenylgruppen, gegebenenfalls funktionell abgewandelte Carboxygruppen oder P und Q sowie P' und Q' zusammen einen ankondensierten Benzolkern,
bedeuten, dadurch gekennzeichnet, daß man Verbindungen der allgemeinen Formel (II)

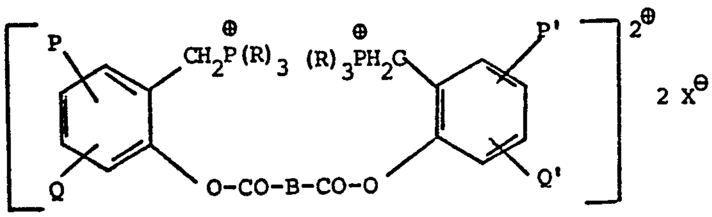

(II)

in welcher
B, P, Q, P', Q' die oben angegebene Bedeutung haben und
R einen Alkyl-, Aryl- oder Aralkylrest und
X ein Anion bedeuten, in durch Alkylgruppen oder Halogenatome substituierten, aromatischen

Kohlenwasserstoffen und in Gegenwart von mindestens 2 Mol Alkali- oder Erdalkalicarbonaten bei einer Temperatur von 100 bis 200 °C unter Abdestillieren des aus den Carbonaten gebildeten Wassers cyclisiert.

2. Verfahren nach Anspruch 1, dadurch gekennzeichnet, daß man als Cyclisierungsmittel Natrium- oder Kaliumcarbonat verwendet.

3. Verfahren nach Anspruch 1, dadurch gekennzeichnet, daß man als aprotisches Lösungsmittel chlorierte Kohlenwasserstoffe wie Chlorbenzol, o-Dichlorbenzol oder Trichlorbenzol verwendet.

4. Verfahren nach Anspruch 1, dadurch gekennzeichnet, daß man die Cyclisierung bei Temperaturen von 130-180° durchführt.

5. Verfahren nach Anspruch 1, dadurch gekennzeichnet, daß man die Cyclisierungsreaktion unter Überleiten eines schwachen Stickstoffstromes durchführt.

**Claims**

1. Process for the manufacture of bis [benzofuranyl-(2)] compounds of the general formula (I)

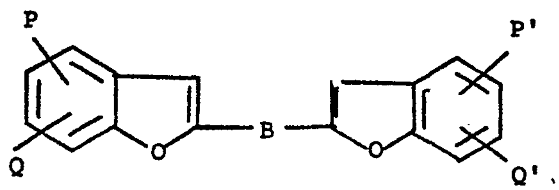

(I)

in which B denotes a direct link or one of the following groups

$-CH=CH-$ , 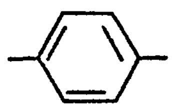 , 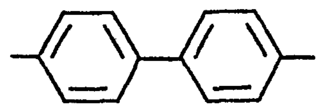 ,

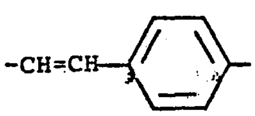 , 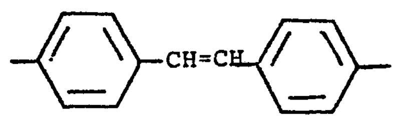 ,

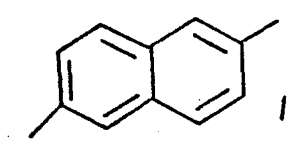 , 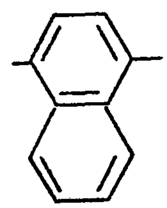 and 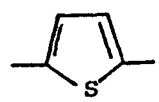

P, Q, P′ and Q′ independent of one another, are hydrogen or halogen atoms, lower alkoxy or phenyl, optionally functionally modified carboxy groups, or P and Q as well P′ and Q′ together denote a fused benzene nucleus, which is characterized by subjecting compounds of the formula II

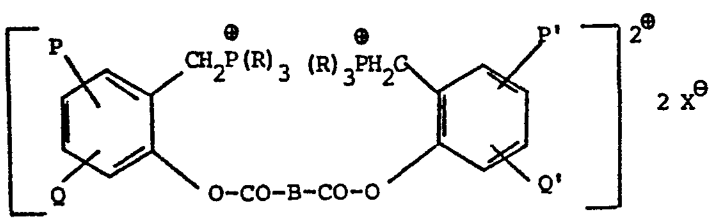

(II)

in which

B, P, Q, P′ and Q′ are as defined above and

R denotes alkyl, aryl or aralkyl and

X stands for an anion, to a cyclization reation in aromatic hydrocarbons substituted by alkyl groups

or halogen atoms and in the presence of at least 2 moles of alkali metal or alkaline earth metal carbonates at a temperature of from 100 to 200 °C while distilling off the water formed from the carbonate.

2. The process of claim 1, wherein sodium carbonate or potassium carbonate is used as cyclization agent.

3. The process of claim 1, wherein chlorinated hydrocarbons such as chlorobenzene, o-dichlorobenzene or trichlorobenzene are used as aprotic solvents.

4. The process of claim 1, wherein the cyclization is carried out at temperatures of from 130 to 180 °C.

5. The process of claim 1, wherein the cyclization is carried out while passing over a weak nitrogen current.

**Revendications**

1. Procédé de préparation de composés bis-[benzofurannyliques-(2)] de formule générale (I)

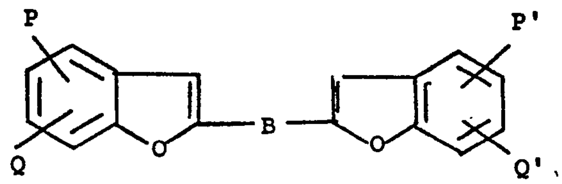

(I)

dans laquelle B représente une liaison directe ou l'un des groupes suivants

$-CH=CH-$ ,

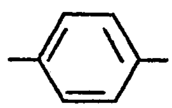

,

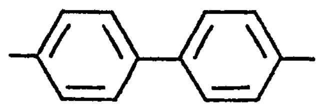

,

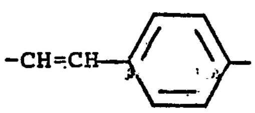

,

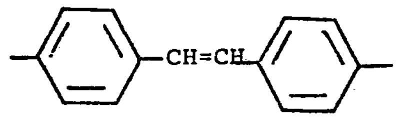

,

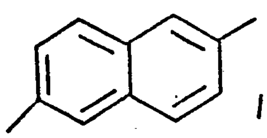

,

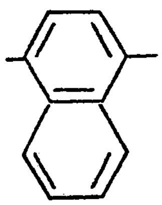

et

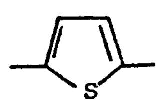

et P, Q, P' et Q' représentent, indépendamment les uns des autres, l'hydrogène ou des atomes d'halogènes, des groupes alcoxy inférieurs ou phényle ou encore des groupes carboxyliques à fonction éventuellement modifiée, ou bien P et Q, ainsi que P' et Q', représentent ensemble un cycle benzénique condensé, procédé caractérisé en ce que l'on cyclise des composés de formule générale (II)

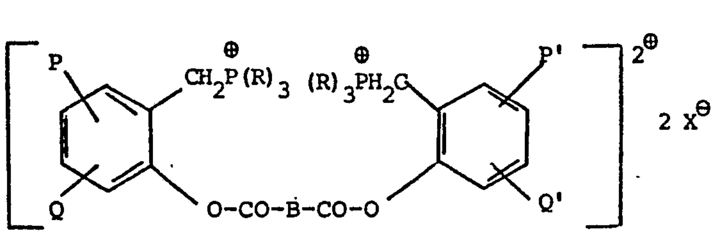

(II)

dans laquelle

les symboles B, P, Q, P' et Q' ont les significations précédentes, et

R représente un radical alkyle, aryle ou aralkyle et

X un anion dans des hydrocarbures aromatiques substitués par des alkyles ou des halogènes et en

présence d'au moins 2 moles de carbonates de métaux alcalins ou alcalino-terreux, à une température de 100 à 200 °C, tout en éliminant par distillation l'eau formée par les carbonates.

2. Procédé selon la revendication 1, caractérisé en ce que l'on utilise comme agent de cyclisation du carbonate de sodium ou de potassium.

3. Procédé selon la revendication 1, caractérisé en ce que l'on utilise comme solvant aprotique un hydrocarbure chloré tel que le chlorobenzène, l'ortho-dichlorobenzène ou le trichlorobenzène.

4. Procédé selon la revendication 1, caractérisé en ce que l'on effectue la cyclisation à des températures de 130 à 180°.

5. Procédé selon la revendication 1, caractérisé en ce que l'on effectue la réaction de cyclisation sous un léger courant d'azote.